# EUROPEAN PATENT SPECIFICATION

(11) **EP 1 035 201 B1**
(45) Date of publication and mention of the grant of the patent: **25.09.2002**
(21) Application number: 00301452.9
(22) Date of filing: 24.02.2000
(51) Int. Cl.: C12M 1/22

(54) **Growth container**
Kulturgefass
Récipient de culture

(30) Priority: 08.03.1999 US 264552
(43) Date of publication of application: 13.09.2000
(73) Proprietor: Anderson, Mark L., Spring Valley, WI 54767 (US)
(72) Inventor: Anderson, Mark L., Spring Valley, Wisconsin (US); Harmon, John C., Eau Galle, Wisconsin (US)
(74) Representative: Higgins, Michael Roger

(56) References cited:
- EP-A- 0 119 984
- EP-A- 0 171 174
- US-A- 3 597 326
- US-A- 4 072 577

## Description

### BACKGROUND OF THE INVENTION

### 1. Field of the Invention.

The present invention relates, generally, to growth containers such as petri dishes. More particularly, the invention relates to petri dishes having a lid that can be oriented and placed on a dish in a number of predetermined positions to either restrict or permit gas exchange between the interior and exterior of the petri dish.

### 2. Background Information.

A petri dish is a small shallow dish of thin glass or plastic with a loose cover that is used to grow tissue cultures and microorganisms on a solid culture. Generally, the lid covers the dish to prevent or minimize contamination during the culturing process. However, there will not be adequate gas exchange if the lid is fully inserted over the dish. Gas exchange is important for the culturing processes, and is especially important for anaerobic culturing processes in which oxygen-containing gas is removed or replaced from the petri dish. Typically, the lid is either completely removed or is only partially inserted over the dish when it is desired to expose the culture to ambient gas. It can be difficult to retain the desired position of a lid partially inserted on the dish when the petri dish is moved. Because large quantities of petri dishes may be used for some processes or experiments, the lid should be able to be quickly, effectively, and accurately placed, removed, and replaced on the dish. Additionally, it may be desirable to stack these petri dishes on top of each other because of space constraints.

The state of the art includes various petri dishes. U.S. Patent No. 5,520,302 discloses a petri dish comprised of a generally rectangular dish and a lid. Two ' adjacent comers of both the dish and the lid are rounded and the other two adjacent corners of both the dish and the lid are notched. The lid has two positions on the dish. In a first position, the notched corners of the lid lie on the rounded comers of the dish and the rounded corners of the lid lie on the notched comers of the dish. The differently-shaped corners prevent the lid from being fully inserted over the dish in this position, allowing gas flow between the dish and the lid. In a second position, the notched corners of the lid are matched with the notched corners of the dish and the rounded corners of the lid are matched with the rounded corners of the dish. In this position, the lid is fully inserted over the dish and severely restricts or prevents gas flow between the lid and-the dish. U.S. Patent No. 4,675,298 discloses a petri dish that has projections formed on the periphery of the inner surface of the cover and has matching depressions on the rim of the lower dish. The projections and depressions provide an adjustable gap between the interior of the petri dish and the atmosphere.

EP-A-0 171 174 discloses a Petri dish having abutments and positioning members located in confronting side walls of the base and the cover lid for locking the dish at different positions, wherein each position admits the entrance of ambient atmosphere into the dish at different rates. EP-A-0 119 984 discloses a Petri dish having position members located on the inner surface of the top wall of the cover lid and corresponding grooves located on an upper surface of the side wall of the base member, such as to allow entrance of ambient atmosphere into the dish when the position members and the grooves are mismatched. These Petri dishes allow a control of the rate of gas exchange with the outer environment depending on the relative position of the cover lid with respect to the base portion by means of a key/keyway mechanismus. US-A-3,597,326 discloses a stackable square-shaped multi-well Petri dish. The construction of this Petri dish does not allow to control the rate of gas exchange.

Applicants' invention provides a petri dish which is believed to constitute an improvement over existing technology.

### BRIEF SUMMARY OF THE INVENTION

The present invention provides an improved container or petri dish for growing tissue cultures and microorganisms. The petri dish generally comprises a base or dish portion covered by a lid portion. The dish portion has a surface and a peripheral dish wall around the surface. The lid portion has a ceiling plate and a peripheral lid wall around the ceiling plate. The lid wall has a complementary shape with respect to the dish wall to retain the lid portion on the dish portion in a number of predetermined or desired positions.

The petri dish has a selector or selection features that an individual may use to quickly, effectively and accurately orient and place the lid portion on the dish portion in one of the desired positions to either restrict or permit a desired gas flow between the dish portion and the lid portion. These selection features may permit a desired gas flow in either a stackable or non-stackable configuration. These selection features include a key / keyway combination and a plurality of notched areas along the edge of one of the walls. The key is preferably a protrusion formed on the interior of the lid wall, and the keyway is preferably a small channel formed in the exterior of an upper wall portion of the dish wall. The lid portion is oriented with respect to and placed on the dish portion to restrict gas flow when the key fits within the keyway because the lid can be fully inserted over the dish. The key rests on the top edge of the dish wall when it is not aligned with the keyway so that the lid is only partially inserted over the dish to form a small vent opening that will permit a desired gas flow in a non-stackable configuration. The complementary shapes of the lid wall and dish wall retain the lid portion on the dish portion.

The notched areas are formed along an edge of one of the walls and contact the edge of the other wall when the lid portion is oriented with respect to and placed on the dish portion to form uniformly spaced and larger vent openings. The lid portion and dish portion form these vent openings in a stackable configuration. These notched areas are preferably formed on the bottom edge of the lid wall. The notched areas have a predetermined vertical dimension and have a predetermined length to span across adjacent sides or segments of the dish wall when the lid portion is rotated with respect to the dish portion. The notches in the lid wall cooperate with the dish wall to retain the lid portion in the desired "rotated" position on the dish portion to permit a desired gas flow. The lid portion rests on the dish portion in a stable or secure manner, allowing other dishes to be stacked on top.

The features, benefits and objects of this invention will become clear to those skilled in the art by reference to the following description, claims and drawings.

### BRIEF DESCRIPTION OF THE SEVERAL VIEWS OF THE DRAWING

Figure 1 is a top plan view of the dish portion of the petri dish of the present invention.

Figure 2 is a bottom plan view of the lid portion of the petri dish of the present invention.

Figure 3 is a side view of the petri dish of the present invention, wherein the lid portion is oriented with respect to and is being placed on the dish portion in a predetermined position to restrict gas flow.

Figure 4 is a side view of the petri dish of Figure 3, wherein the lid portion is placed on the dish portion in a predetermined position to restrict gas flow.

Figure 5 is a side view of the petri dish of the present invention, wherein the lid portion is oriented with respect to and is being placed on the dish portion in a predetermined position to permit gas flow in a non-stackable configuration.

Figure 6 is a side view of the petri dish of Figure 5, wherein the lid portion is placed on the dish portion and the key of the lid portion rests on the top edge of the dish wall in a non-stackable configuration.

Figure 7 is a top plan view of the petri dish of the present invention in a predetermined position to permit gas flow in a stackable configuration, wherein the lid portion is rotated an offset amount and the notched areas of the lid portion span across adjacent dish walls.

Figure 8 is a side plan view of the petri dish of Figure 7.

### DETAILED DESCRIPTION

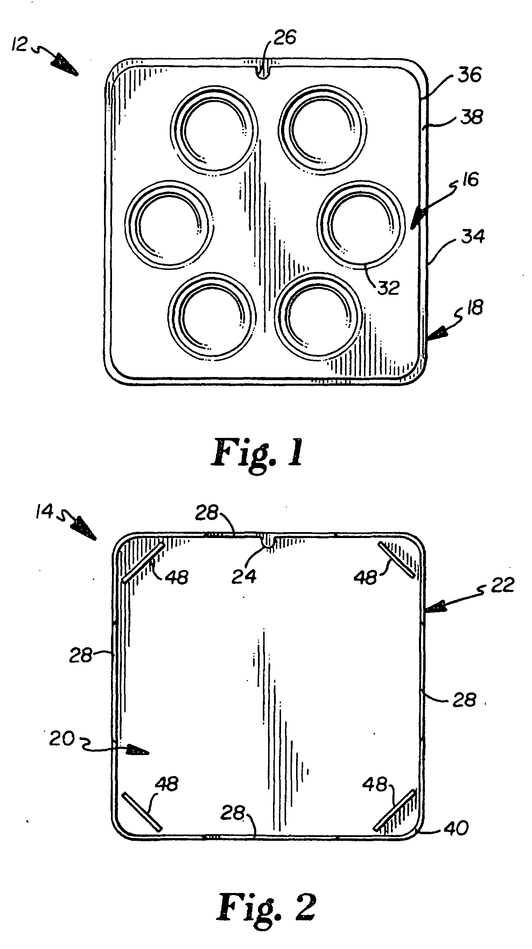

Referring to **Figures 1-8,** an example of the preferred embodiment of the present invention is illustrated and generally indicated by the reference numeral 10. The petri dish 10 is described below first in terms of its major structural elements and then in terms of its secondary structural and/or functional elements which cooperate to provide an economical petri dish that an individual can quickly, effectively, and accurately orient and place, remove, and replace a lid portion on a dish portion in a desired position to either restrict or permit a desired gas flow.

The container or petri dish 10 generally comprises a base or dish portion 12 covered by a lid portion 14. The dish portion 12 has a surface 16 and a peripheral base or dish wall 18 around the surface 16, and the lid portion 14 has a ceiling plate 20 and a peripheral lid wall 22 around the ceiling plate 20. The lid wall 22 and the dish wall 18 have complementary shapes with respect to each other. The shapes of these walls 18 and 22 retain the lid portion 14 on the dish portion 12 in a number of predetermined positions to either restrict or permit a desired gas flow between the interior and exterior of the petri dish 10.

The petri dish 10 has a selector or selection features that help an individual orient and place the lid portion 14 on the dish portion 12 in one of the desired positions to either restrict or permit a desired gas flow between the dish portion 12 and the lid portion 14. These features include a key 24 and a keyway 26 and a plurality of notched areas 28 in one of the walls.

The petri dish 10 is preferably constructed or formed from a clear plastic. However, glass or other materials may be used. The surface 16 of the dish portion 12 is preferably a raised surface extending from the top edge 30 of the dish wall 18. The raised surface 16 may have a plurality of recessed wells 32 in which separate and distinct cultures may be grown within same petri dish 10. Preferably the dish wall 18 is an endless wall that extends around the periphery of the surface 16, and comprises a lower wall portion 34 and an upper wall portion 36. The lower wall portion 34 has a perimeter greater than the upper wall portion 36 and forms a lip 38 between the two wall portions. The lid wall 22 of the lid portion 14 is preferably an endless wall that extends down from the periphery of the ceiling plate 20. The bottom edge 40 of the lid wall 22 abuts against the lip 38 of the dish wall 18 when the lid portion 14 is fully inserted over the dish portion 12 to restrict gas flow. In a preferred embodiment shown in the figures, the petri dish 10 is about 7.62 cm (3 inches) long, 7.62 cm (3 inches) wide, and 1.27 cm (½ inch) high. The petri dish 10 has six recessed wells 32 positioned in a circular pattern in the dish portion 12. The wells 32 are approximately 1.27 cm (½ inch) deep and have a diameter of approximately 1.905 cm (¾ inch) The top surface of the top or ceiling plate 20 preferably has ridges 48 that cooperate with the dish wall 18 of a stacked petri dish 10. The ridges 48 promote proper alignment between the lid portions and dish portions of stacked petri dishes, and prevent a stacked petri dish from slipping of the lid of the bottom petri dish.

The figures show the key 24 and keyway 26 formed in the lid portion 14 and the dish portion 12, respectively. The key 24 is preferably a small protrusion, 0.159 cm (1/16 inch) in the illustrated embodiment, formed on the interior of the lid wall 22, and the keyway 26 is preferably a small channel formed in the exterior of the dish wall 18 and sized to receive the key 24. However, the key 24 could be formed in the dish portion 12 and the keyway 26 could be formed in the lid portion 14.

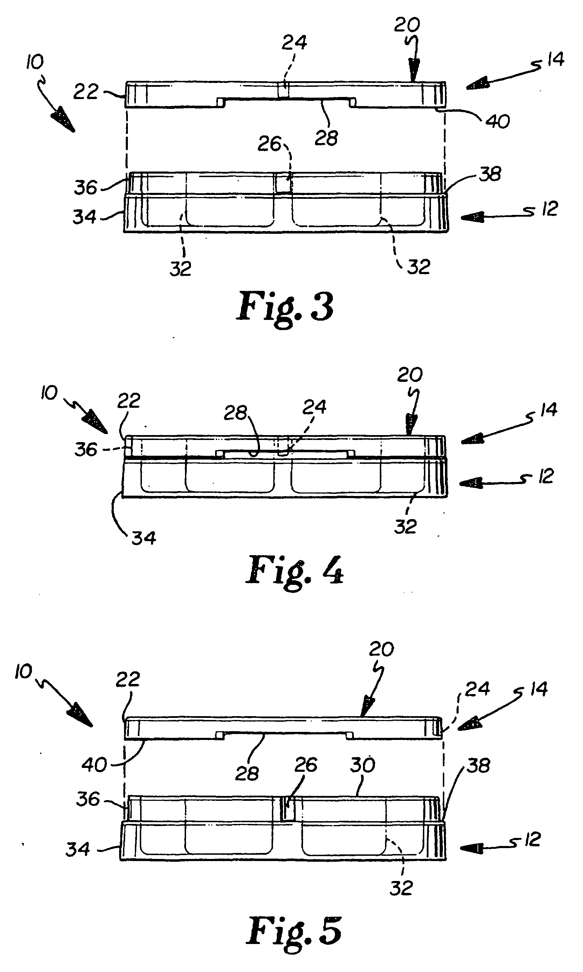

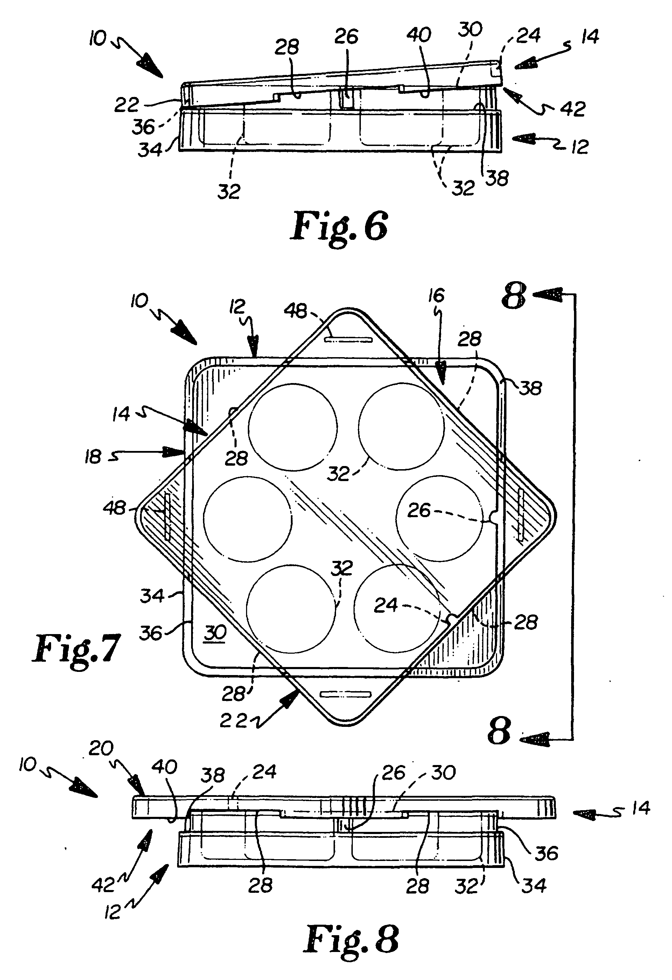

In a preferred embodiment shown in the figures, the lid portion 14 and dish portion 12 of the petri dish 10 have a generally square configuration or footprint that allow the lid portion 14 to fit over the dish portion 12 at four rotational positions (0°, 90°, 180°, and 270°) with respect to each other. The corners of the lid portion 14 and dish portion 12 are preferably rounded. Other symmetrical shapes that have adjacent sides, such as triangles or pentagons, would function similarly to the illustrated embodiment as described below. Referring to **Figures 3-4,** the lid portion 14 is ' oriented with respect to and placed on the dish portion 12 at 0°, for example, where the key 24 fits within the keyway 26. Gas flow is restricted in this position because the lid portion 14 can be fully inserted over the dish portion 12 where the bottom edge 40 of the lid wall 22 abuts against the lip 38 of the dish wall 18. The key 24 is not aligned with the keyway 26 when the lid portion 14 is oriented with respect to and placed on the dish portion 12 at 90°, 180°, or 270°. In these positions, as illustrated in **Figures 5-6**, the key 24 rests on the top edge 30 of the dish wall 18. A relatively small vent opening 42 is formed to permit a desired gas flow. On the opposite side of the lid portion 14, the bottom edge 40 of the lid wall 22 abuts against the lip 38 of the dish wall 18. The remainder of the lid wall 22 retains the lid portion 14 on the dish portion 12 in the desired position because, as shown in **Figures 3** and **6**, the bottom of the key 24 is higher than the bottom edge 40 of the lid wall 22. Multiple keys 24 and / or multiple keyways 26 may be used to provide additional positions where the lid portion 12 would be fully inserted over the dish portion 12 to restrict gas flow. The petri dishes are not stackable in this configuration.

The notched areas 28 are formed on one of the walls and contact the other wall when the lid portion 14 is oriented with respect to and placed on the dish portion 12 to form uniformly spaced and larger vent openings 42 that will permit a desired gas flow. As illustrated in the bottom plan view of the lid wall 22 shown in **Figure 2,** the notched areas 12 are formed on the bottom edge 40 of the lid wall 22 and contact the top edge 30 of the dish wall 18 in a preferred embodiment. However, the notched areas 12 could be formed in the top edge 30 of the dish wall 18 and contact the bottom edge 40 of the lid wall 22, or both walls 18 and 22 could be formed with notched areas 12 on their respective edges. The notched areas 28 have a predetermined vertical dimension and, as shown in **Figures 7-8**, have a predetermined length to span across adjacent sides or segments of the dish wall 18 when the lid portion 14 is rotated with respect to the dish portion 12. For the generally square petri dish 10 illustrated in the figures, the notched areas 28 in the lid wall 22 extend across adjacent sides or segments of the dish wall 18 when the lid portion 14 is oriented at about 45°, 135°, 225°, and 315° with respect to the dish portion. A notched area 28 is generally centered along each side of the lid wall 22, and each notch area has a length of approximately 1 1/8 inch in the illustrated embodiment. Other symmetrical shapes that have adjacent sides, such as a triangle, pentagon, etc., would function similar to the illustrated embodiment as described below. The lid wall 22 retains the lid portion 14 on the dish portion 12 in the desired "rotated" position to form vent openings 42 that will permit a desired gas flow. The positioning of the lid portion 14 illustrated in **Figure 8** provides vent openings 42 that are uniformly spaced and are relatively larger than the vent openings formed by the positioning of the lid portion 14 illustrated in **Figure 6**. The petri dishes are stackable in this configuration.

## Claims

1. A petri-dish (10) comprising:
(a) a base portion (12) having a bottom surface (16) and a peripheral wall (18) around said surface (16):
(b) a lid portion (14) having a top plate (20) and a peripheral wall (22) around said top plate (20), said lid wall (22) having a complementary shape with respect to said base wall (18) to retain said lid portion (14) on said base portion (12) in a plurality of predetermined positions; and
(c) a selector for orientating and placing said lid portion (14) on said base portion (12) in one of said positions to either restrict or permit gas flow between said base portion (12) and said lid portion (14), **characterised in that** the selector comprises a keyway (26) in the peripheral wall (18) of the base portion (12), a plurality of notched areas (28) formed in the peripheral wall (22) of the lid portion (14) for permitting gas flow between said base portion (12) and said lid portion (14) when said lid portion (14) is orientated with respect to and placed on said base portion (12) in a first position and a key (24) formed by said lid wall (22) for fitting within the keyway (26) when the lid portion (14) is orientated with respect to and placed on said base portion (12) in a second position to restrict gas flow.

2. The petri-dish of claim 1, wherein said base wall (18) includes a lower wall (34) separated from an upper wall (36) by a lip (38), said lower wall (34) having a perimeter greater than said upper wall (36) and approximately equal to said lid wall (22), said lid wall (22) extending around said upper wall (36) and abutting against said lip (38) when said lid portion (14) covers said base portion (12) to restrict gas flow between said base portion (12) and said lid portion (14).

3. The petri-dish of claim 1 or claim 2, wherein said key (24) is not aligned with said keyway (26) and rests on the top edge (30) of said peripheral wall (18) of the base portion (12) when said lid portion (14) is orientated with respect to and placed on said base portion (12) in at least one of said positions to permit gas flow.

4. The petri-dish of claim 3, wherein said base portion (12) and said lid portion (14) are in a non-stackable configuration when said key (24) is not aligned with said keyway (26).

5. The petri-dish according to any one of the preceding claims, wherein said bottom surface (16) is a raised planar surface extending from the top edge (30) of said base wall (18), said raised surface (16) containing the at least one recessed well (32).

6. The petri-dish of any one of the preceding claims, wherein said notched areas (28) contact the top edge (30) of said base wall (18) when said lid portion (14) is orientated with respect to and placed on said base portion (12) in said first position to permit a desired gas flow.

7. The petri-dish of claim 6, wherein said base wall (18) has sides and wherein said notched areas (28) have a predetermined vertical dimension compared to the remainder of said lid wall (22) and have a predetermined length to span across adjacent sides of said base wall (18) when said lid portion (14) is orientated with respect to and placed on said base portion (12) to permit a desired gas flow.

8. The petri-dish of claim 6 or claim 7, wherein said lid wall (22) and said base wall (18) have a generally square configuration comprised of four sides, said lid wall (22) having four notched areas (28), each of said notched areas (28) being generally centred along one of said sides of said lid wall (22).

9. The petri-dish of any one of the preceding claims, wherein said base portion (12) and said lid portion (14) are in a stackable configuration when said notched areas (28) contact the peripheral wall (18) of the base portion (12).

10. The petri-dish according to any one of the preceding claims, wherein said top plate (20) has a top surface with ridges (48) positioned and arranged to cooperate with said peripheral wall (18) of said base portion (12) of a stacked container, said ridges (48) preventing said stacked container from slipping.

11. A petri-dish (10) comprising:
(a) a base portion (12) having a surface (16) and a peripheral base wall (18) around said surface (16),
(i) said base wall (18) including a lower wall (34) separated from an upper wall (36) by a lip (38), said lower wall (34) having a perimeter greater than said upper wall (36) and approximately equal to said lid wall (22), said lid wall (22) extending around said upper wall (36) and abutting against said lip (38) when said lid portion (14) is orientated with respect to and placed on said base portion (12) to restrict gas flow between said base portion (12) and said lid portion (14),
(ii) said base wall (18) having a keyway notch (26) orientated outward from said base wall (18), and
(iii) said surface (16) being a raised planar surface extending from a top edge (30) of said base wall (18), said raised surface (16) containing at least one recessed well (32); and
(b) a lid portion (14) having a ceiling plate (20) and a peripheral lid wall (22) around said ceiling plate (20), said lid wall (22) having a complementary shape with respect to said base wall (18) to retain said lid portion (14) on said base portion (12) in a number of predetermined positions,
(i) both said lid wall (22) and base wall (18) having a generally square configuration comprised of four sides,
(ii) said lid wall (22) having a notched area (28) on each side of said lid wall (22), each of said notched areas (28) having a shorter predetermined vertical dimension compared to the remainder of said lid wall (22), each of said notched areas (28) being generally centred along one of said sides of said lid wall (22), said notched areas (28) of said lid (14) resting on a top edge (30) of said base wall (18) when said lid (14) is orientated with respect to and placed on said base portion (12) in one of said positions to permit gas flow, each of said notched areas (28) having a predetermined length and spanning across adjacent sides of said base wall (18) when said lid portion (14) is orientated with respect to and placed on said base portion (12) in one of said positions to permit gas flow,
(iii) said lid wall (22) having a key (24) formed in said lid portion (14), said key (24) being received by said keyway notch (26) when said lid portion (14) is orientated with respect to and placed on said base portion (12) in one of said positions to restrict gas flow, said key (24) resting on a top edge (30) of said base wall (18) when said lid portion (14) is orientated with respect to and placed on said base portion (12) in one of said positions to permit gas flow, and
(iv) said ceiling plate (20) having a top surface with ridges (48) positioned and arranged to cooperate with said base wall (18) of said base portion (12) for a stacked container, said ridges (48) preventing said stacked container from slipping.

## Patentansprüche

1. Petrischale (10), die folgendes umfaßt:
(a) einen Basisteil (12) mit einer Bodenfläche (16) und einer Umfangswand (18) um die Fläche (16),
(b) einen Deckelteil (14) mit einer Deckplatte (20) und einer Umfangswand (22) um die Deckplatte (20), wobei die Deckelwand (22) eine komplementäre Form in Bezug auf die Basiswand (18) hat, um den Deckelteil (14) auf dem Basisteil (12) in einer Vielzahl von vorher festgelegten Positionen zu halten, und
(c) eine Auswahlvorrichtung zum Ausrichten und Positionieren des Deckelteils (14) auf dem Basisteil (12) in einer der Positionen, um einen Gasfluß zwischen dem Basisteil (12) und dem Deckelteil (14) entweder zu beschränken oder zu ermöglichen, **dadurch gekennzeichnet, daß** die Auswahlvorrichtung eine Keilnut (26) in der Umfangswand (18) des Basisteils (12), eine Vielzahl von gekerbten Bereichen (28), die in der Umfangswand (22) des Deckelteils (14) gebildet werden, um einen Gasfluß zwischen dem Basisteil (12) und dem Deckelteil (14) zu ermöglichen, wenn der Deckelteil (14) in einer ersten Position in Bezug auf den Basisteil (12) ausgerichtet und auf demselben positioniert ist, und einen Keil (24) umfaßt, der durch die Deckelwand (22) gebildet wird, um in die Keilnut (26) zu passen, wenn der Deckelteil (14) in einer zweiten Position in Bezug auf den Basisteil (12) ausgerichtet und auf demselben positioniert ist, um einen Gasfluß zu beschränken.

2. Petrischale nach Anspruch 1, bei der die Basiswand (18) eine untere Wand (34) einschließt, die durch einen Rand (38) von einer oberen Wand (36) getrennt wird, wobei die untere Wand (34) einen Umfang hat, der größer ist als derjenige der oberen Wand (36) und annähernd gleich groß wie derjenige der Deckelwand (22), wobei die Deckelwand (22) um die obere Wand (36) verläuft und an den Rand (38) anstößt, wenn der Deckelteil (14) den Basisteil (12) bedeckt, um einen Gasfluß zwischen dem Basisteil (12) und dem Deckelteil (14) zu beschränken.

3. Petrischale nach Anspruch 1 oder Anspruch 2, bei welcher der Keil (24) nicht mit der Keilnut (26) ausgerichtet ist und auf der Oberkante (30) der Umfangswand (18) des Basisteils (12) ruht, wenn der Deckelteil (14) in wenigstens einer der Positionen in Bezug auf den Basisteil (12) ausgerichtet und auf demselben positioniert ist, um einen Gasfluß zu ermöglichen.

4. Petrischale nach Anspruch 3, bei welcher der Basisteil (12) und der Deckelteil (14) in einer nicht-stapelbaren Konfiguration sind, wenn der Keil (24) nicht mit der Keilnut (26) ausgerichtet ist.

5. Petrischale nach einem der vorhergehenden Ansprüche, bei der die Bodenfläche (16) eine erhöhte ebene Fläche ist, die von der Oberkante (30) der Basiswand (18) vorsteht, wobei die erhöhte Fläche (16) wenigstens einen ausgesparten Schacht (32) enthält.

6. Petrischale nach einem der vorhergehenden Ansprüche, bei der die gekerbten Bereiche (28) die Oberkante (30) der Basiswand (18) kontaktieren, wenn der Deckelteil (14) in der ersten Position in Bezug auf den Basisteil (12) ausgerichtet und auf demselben positioniert ist, um einen gewünschten Gasfluß zu ermöglichen.

7. Petrischale nach Anspruch 6, bei der die Basiswand (18) Seiten hat und bei der die gekerbten Bereiche (28) eine vorher festgelegte vertikale Abmessung im Vergleich mit dem Rest der Deckelwand (22) haben und eine vorher festgelegte Länge haben, um sich über aneinandergrenzende Seiten der Basiswand (18) zu erstrecken, wenn der Deckelteil (14) in Bezug auf den Basisteil (12) ausgerichtet und auf demselben positioniert ist, um einen gewünschten Gasfluß zu ermöglichen.

8. Petrischale nach Anspruch 6 oder Anspruch 7, bei der die Deckelwand (22) und die Basiswand (18) eine allgemein quadratische Konfiguration haben, die aus vier Seiten besteht, wobei die Deckelwand (22) vier gekerbte Bereiche (28) hat, wobei sich jeder der gekerbten Bereiche (28) allgemein mittig längs einer der Seiten der Deckelwand (22) befindet.

9. Petrischale nach einem der vorhergehenden Ansprüche, bei welcher der Basisteil (12) und der Deckelteil (14) in einer stapelbaren Konfiguration sind, wenn die gekerbten Bereiche (28) die Umfangswand (18) des Basisteils (12) kontaktieren.

10. Petrischale nach einem der vorhergehenden Ansprüche, bei der die Deckplatte (20) eine Deckfläche mit Stegen (48) hat, die positioniert und angeordnet sind, um mit der Umfangswand (18) des Basisteils (12) eines gestapelten Behälters zusammenzuwirken, wobei die Stege (48) ein Rutschen des gestapelten Behälters verhindern.

11. Petrischale (10), die folgendes umfaßt:
(a) einen Basisteil (12) mit einer Fläche (16) und einer Umfangswand (18) um die Fläche (16),
(i) wobei die Basiswand (18) eine untere Wand (34) einschließt, die durch einen Rand (38) von einer oberen Wand (36) getrennt wird, wobei die untere Wand (34) einen Umfang hat, der größer ist als derjenige der oberen Wand (36) und annähernd gleich groß wie derjenige der Deckelwand (22), wobei die Deckelwand (22) um die obere Wand (36) verläuft und an den Rand (38) anstößt, wenn der Deckelteil (14) in Bezug auf den Basisteil (12) ausgerichtet und auf demselben positioniert ist, um einen Gasfluß zwischen dem Basisteil (12) und dem Deckelteil (14) zu beschränken,
(ii) wobei die Basiswand (18) eine Keilnutkerbe (26) hat, die von der Basiswand (18) nach außen gerichtet ist, und
(iii) wobei die Fläche (16) eine erhöhte ebene Fläche ist, die von einer Oberkante (30) der Basiswand (18) vorsteht, wobei die erhöhte Fläche (16) wenigstens einen ausgesparten Schacht (32) enthält, und
(b) einen Deckelteil (14) mit einer Deckplatte (20) und einer Umfangsdeckelwand (22) um die Deckplatte (20), wobei die Deckelwand (22) eine komplementäre Form in Bezug auf die Basiswand (18) hat, um den Deckelteil (14) auf dem Basisteil (12) in einer Zahl von vorher festgelegten Positionen zu halten,
(i) wobei sowohl die Deckelwand (22) als auch die Basiswand (18) eine allgemein quadratische Konfiguration haben, die aus vier Seiten besteht,
(ii) wobei die Deckelwand (22) einen gekerbten Bereich (28) auf jeder Seite der Deckelwand (22) hat, wobei jeder der gekerbten Bereiche (28) eine kürzere vorher festgelegte vertikale Abmessung im Vergleich mit dem Rest der Deckelwand (22) hat, wobei sich jeder der gekerbten Bereiche (28) allgemein mittig längs einer der Seiten der Deckelwand (22) befindet, wobei die gekerbten Bereiche (28) des Deckels (14) auf einer Oberkante (30) der Basiswand (18) ruhen, wenn der Deckel (14) in einer der Positionen in Bezug auf den Basisteil (12) ausgerichtet und auf demselben positioniert ist, um einen Gasfluß zu ermöglichen, wobei jeder der gekerbten Bereiche (28) eine vorher festgelegte Länge hat und sich über aneinandergrenzende Seiten der Basiswand (18) erstreckt, wenn der Deckelteil (14) in einer der Positionen in Bezug auf den Basisteil (12) ausgerichtet und auf demselben positioniert ist, um einen Gasfluß zu ermöglichen,
(iii) wobei die Deckelwand (22) einen Keil (24) hat, der in dem Deckelteil (14) gebildet wird, wobei der Keil (24) durch die Keilnutkerbe (26) aufgenommen wird, wenn der Deckelteil (14) in einer der Positionen in Bezug auf den Basisteil (12) ausgerichtet und auf demselben positioniert ist, um einen Gasfluß zu beschränken, wobei der Keil (24) auf einer Oberkante (30) der Basiswand (18) ruht, wenn der Deckelteil (14) in einer der Positionen in Bezug auf den Basisteil (12) ausgerichtet und auf demselben positioniert ist, um einen Gasfluß zu ermöglichen, und
(iv) wobei die Deckplatte (20) eine Deckfläche mit Stegen (48) hat, die positioniert und angeordnet sind, um mit der Basiswand (18) des Basisteils (12) eines gestapelten Behälters zusammenzuwirken, wobei die Stege (48) ein Rutschen des gestapelten Behälters verhindern.

## Revendications

1. Boîte de Pétri (10), comprenant:
(a) une partie de base (12) comportant une surface inférieure (16) et une paroi périphérique (18) entourant ladite surface (16);
(b) une partie de couvercle (14) comportant une plaque supérieure (20) et une paroi périphérique (22) entourant ladite plaque supérieure (20), ladite paroi du couvercle (22) ayant une forme complémentaire de ladite paroi de base (18) pour retenir ladite partie de couvercle (14) sur ladite partie de base (12) dans plusieurs positions prédéterminées; et
(c) un sélecteur pour orienter et placer ladite partie de couvercle (14) sur ladite partie de base (12) dans une desdites positions, pour restreindre ou permettre l'écoulement de gaz entre ladite partie de base (12) et ladite partie de couvercle (14), **caractérisée en ce que** le sélecteur comprend une rainure de clavette (26) dans la paroi périphérique (18) de la partie de base (12), plusieurs zones entaillées (28) étant formées dans la paroi périphérique (22) de la partie de couvercle (14) pour permettre l'écoulement de gaz entre ladite partie de base (12) et ladite partie de couvercle (14) lorsque ladite partie de couvercle (14) est orientée par rapport à ladite partie de base (12) et placée sur celle-ci dans une première position, une clavette (24) étant formée par ladite paroi du couvercle (22) destinée à être ajustée dans la rainure de clavette (26) lorsque la partie de couvercle (14) est orientée par rapport à ladite partie de base (12) et placée sur elle-ci dans une deuxième position pour restreindre l'écoulement de gaz.

2. Boîte de Pétri selon la revendication 1, dans laquelle ladite paroi de base (18) englobe une paroi inférieure (34) séparée d'une paroi supérieure (36) par un rebord (38), ladite paroi inférieure (34) ayant un périmètre supérieur à ladite paroi supérieure (34) et à peu près égal à ladite paroi du couvercle (22), ladite paroi du couvercle (22) s'étendant autour de ladite paroi supérieure (36) et butant contre ledit rebord (38) lorsque ladite partie de couvercle (14) recouvre ladite partie de base (12), pour restreindre l'écoulement de gaz entre ladite partie de base (12) et ladite partie de couvercle (14).

3. Boîte de Pétri selon les revendications 1 ou 2, dans laquelle ladite clavette (24) n'est pas alignée avec ladite rainure de clavette (26) et repose sur le bord supérieur (30) de ladite paroi périphérique (18) de la partie de base (12) lorsque ladite partie de couvercle (14) est orientée par rapport à ladite partie de base (12) et placée sur celle-ci dans au moins une desdites positions pour permettre l'écoulement de gaz.

4. Boîte de Pétri selon la revendication 3, dans laquelle ladite partie de base (12) et ladite partie de couvercle (14) ont une configuration non empilable lorsque ladite clavette (24) n'est pas alignée avec ladite rainure de clavette (26).

5. Boîte de Pétri selon l'une quelconque des revendications précédentes, dans laquelle ladite surface inférieure (16) constitue une surface plane surélevée s'étendant à partir du bord supérieur (30) de ladite paroi de base (18), ladite surface surélevée (16) contenant au moins un puits évidé (32).

6. Boîte de Pétri selon l'une quelconque des revendications précédentes, dans laquelle lesdites zones entaillées (28) contactent le bord supérieur (30) de ladite paroi de base (18) lorsque ladite partie de couvercle (14) est orientée par rapport à ladite partie de base (12) et placée sur celle-ci dans ladite première position pour permettre un écoulement voulu de gaz.

7. Boîte de Pétri selon la revendication 6, dans laquelle ladite paroi de base (18) comporte des côtés, lesdites zones entaillées (28) ayant une dimension verticale prédéterminée par rapport à la partie restante de ladite paroi du couvercle (22) et ayant une longueur prédéterminée pour s'étendre à travers les côtés adjacents de ladite paroi de base (18) lorsque ladite partie de couvercle (14) est orientée par rapport à ladite partie de base (12) et placée sur celle-ci de sorte à permettre un écoulement voulu de gaz.

8. Boîte de Pétri selon les revendications 6 ou 7, dans laquelle ladite paroi du couvercle (22) et ladite paroi de base (18) ont une configuration généralement carrée composée de quatre côtés, ladite paroi du couvercle (22) comportant quatre zones entaillées (28), chacune desdites zones entaillées (28) étant en général centrée le long d'un desdits côtés de ladite paroi du couvercle (22).

9. Boîte de Pétri selon l'une quelconque des revendications précédentes, dans laquelle ladite partie de base (12) et ladite partie de couvercle (14) ont une configuration empilable lorsque lesdites zones entaillées (28) contactent la paroi périphérique (18) de ladite partie de base (12).

10. Boîte de Pétri selon l'une quelconque des revendications précédentes, dans laquelle ladite plaque supérieure (20) comporte une surface supérieure avec des nervures (48) positionnées et agencées de sorte à coopérer avec ladite paroi périphérique (18) de ladite partie de base (12) d'un récipient empilé, lesdites nervures (48) empêchant un glissement dudit récipient empilé.

11. Boite de Pétri (10), comprenant:
(a) une partie de base (12) comportant une surface (16) et une paroi de base périphérique (18) entourant ladite surface (16);
(i) ladite paroi de base (18) englobant une paroi inférieure (34) séparée d'une paroi supérieure (36) par un rebord (38), ladite paroi inférieure (34) ayant un périmètre supérieur à ladite paroi supérieure (36) et à peu près égal à ladite paroi du couvercle (22), ladite paroi du couvercle (22) s'étendant autour de ladite paroi supérieure (36) et butant contre ledit rebord (38) lorsque ladite partie de couvercle (14) est orientée par rapport à ladite partie de base (12) et placée sur celle-ci de sorte à restreindre l'écoulement de gaz entre ladite partie de base (12) et ladite partie de couvercle (14),
(ii) ladite paroi de base (18) comportant une entaille à rainure de clavette (26), orientée vers l'extérieur de ladite paroi de base (18), et
(iii) ladite surface (16) étant constituée par une surface plane surélevée s'étendant à partir d'un bord supérieur (30) de ladite paroi de base (18), ladite surface surélevée (16) contenant au moins un puits évidé (32); et
(b) une partie de couvercle (14) comportant une plaque de plafond (20) et une paroi de couvercle périphérique (22) entourant ladite plaque de plafond (20), ladite paroi du couvercle (22) ayant une forme complémentaire de ladite paroi de base (18) pour retenir ladite partie de couvercle (14) sur ladite partie de base (12) dans plusieurs positions prédéterminées,
(i) ladite paroi du couvercle (22) et ladite paroi de base (18) ayant une configuration généralement carrée composée de quatre côtés,
(ii) ladite paroi du couvercle (22) comportant une zone entaillée (28) de chaque côté de ladite paroi du couvercle (22), chacune desdites zones entaillées (28) ayant une dimension verticale prédéterminée plus courte que la partie restante de ladite paroi du couvercle (22), chacune desdites zones entaillées (28) étant en général centrée le long d'un desdits côtés de ladite paroi du couvercle (22), lesdites zones entaillées (28) dudit couvercle (14) reposant sur un bord supérieur (30) de ladite paroi de base (18) lorsque ledit couvercle (14) est orienté par rapport à ladite partie de base (12) et placé sur celle-ci dans une desdites positions pour permettre l'écoulement de gaz, chacune desdites zones entaillées (28) ayant une longueur prédéterminée et s'étendant à travers les côtés adjacents de ladite paroi de base (18) lorsque ladite partie de couvercle (14) est orientée par rapport à ladite partie de base (12) et placée sur celle-ci dans une desdites positions pour permettre l'écoulement de gaz,
(iii) ladite paroi du couvercle (22) comportant une clavette (24) formée dans ladite partie de couvercle (14), ladite clavette (24) étant reçue par ladite entaille à rainure de clavette (26) lorsque ladite partie de couvercle (14) est orientée par rapport à ladite partie de base (12) et placée sur celle-ci dans une desdites positions pour restreindre l'écoulement de gaz, ladite clavette (24) reposant sur un bord supérieur (30) de ladite paroi de base (18) lorsque ladite partie de couvercle (14) et orientée par rapport à ladite partie de base (12) et placée sur celle-ci dans une desdits positions pour permettre l'écoulement de gaz, et
(iv) ladite plaque de plafond (20) comportant une surface supérieure avec des nervures (48), positionnées et agencées de sorte à coopérer avec ladite paroi de base (18) de ladite partie de base (12) pour former un réservoir empilé, lesdites nervures (48) empêchant un glissement dudit récipient empilé.
